(19) Europäisches Patentamt
European Patent Office
Office européen des brevets

(11) Numéro de publication : **0 488 834 A1**

# (12) DEMANDE DE BREVET EUROPEEN

(21) Numéro de dépôt : **91402848.5**

(22) Date de dépôt : **24.10.91**

(51) Int. Cl.[5] : **C07D 303/04,** C07D 493/10, // (C07D493/10, 303:00, 303:00)

(30) Priorité : **25.10.90 FR 9013250**

(43) Date de publication de la demande : **03.06.92 Bulletin 92/23**

(84) Etats contractants désignés : **AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy les Lyon (FR)**
Inventeur : **Delmond, Bernard**
**12 allée des Bergeronnettes**
**F-33600 Pessac (FR)**
Inventeur : **Filliatre, Claude**
**32 rue Georges Bizet**
**F-33400 Talence (FR)**
Inventeur : **Pereyre, Michel**
**45 rue du Haut Carré**
**F-33400 Talence (FR)**
Inventeur : **Serramedan, Dominique**
**37 rue Réaumur**
**F-17000 La Rochelle (FR)**

(74) Mandataire : **Savina, Jacques et al**
**RHONE-POULENC RORER S.A., Direction des Brevets, 90 avenue Raymond Aron**
**F-92165 Antony Cédex (FR)**

## (54) Dérivés de spiro-époxy-cyclohexane, leur préparation et leur utilisation en chimie des polymères.

(57) La présente invention concerne de nouveaux époxydes de formule générale :

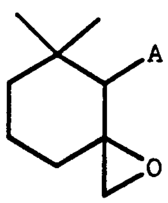

dans laquelle A représente un groupement méthylène ou oxirane ; leur procédé de préparation et leur utilisation.

EP 0 488 834 A1

La présente invention concerne de nouveaux époxydes, leur procédé de préparation et leur utilisation.

Plus particulièrement, les époxydes de l'invention ont pour formule générale (1) :

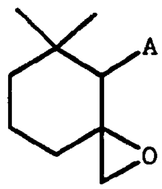

(1)

dans laquelle A représente un groupement méthylène (1a) ou oxirane (1b).

Les composés de l'invention sont donc le :

– Diméthyl-5,5 méthylène-4 oxa-1 spiro(2,5) octane (1a), et le

– Diméthyl-7,7 dioxa-1,5 dispiro(2,0,2,4) décane (1b).

Ces époxydes nouveaux peuvent être utilisés comme stabilisants pour PVC. Ils peuvent également être utilisés comme matière première pour la synthèse de résines époxy, ou en tant que comonomères pour l'obtention d'agents d'adhérence dans les polymères.

On connait dans l'art antérieur des époxydes positionnés sur des structures cycliques similaires. En particulier, ErhenFreund et Coll. (Helv. Chim. Acta., 57(4), 1098 (1974)) ont décrit l'époxyde suivant (2a):

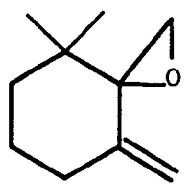

(2a)

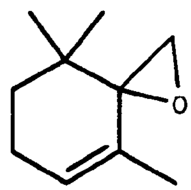

(2b)

obtenu comme sous-produit de l'oxydation de β-cyclogéraniol par le tétraacétate de plomb.

Par ailleurs, Rosenberger et Coll. (Helv. Chim. Acta., 63(6), 1665 (1980) ont décrit l'isomère de position de la double liaison (2b), obtenu par réaction de la triméthyl-2,2,6 cyclohexenone avec un ylure de soufre.

Aucun document antérieur ne décrit ni ne suggère les époxydes de l'invention. Aucun de ces documents ne suggère non plus de voie d'accès à ces composés.

Les composés de formule (1) de l'invention sont préparés par époxydation du pyronène de formule (3) :

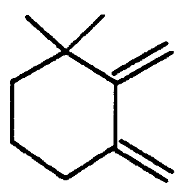

(3)

au moyen d'un agent époxydant.

Plus particulièrement, l'époxydation est réalisée au moyen d'un agent époxydant qui peut être choisi parmi les peracides ou leurs dérivés, les acides hypohalogéneux ou leurs précurseurs, l'eau oxygénée, les hydroperoxydes d'alkyles, les perborates ou les percarbonates.

Parmi les peracides organiques utilisables dans la présente invention, on peut citer les acides et les dérivés d'acides carboxyliques, aliphatiques ou aromatiques, éventuellement substitués. Notamment, l'acide peracétique, l'acide performique, l'acide perpropionique, l'acide pertrifluoroacétique, l'acide paranitroperbenzoïque ou l'acide métachloroperbenzoïque peuvent être employés.

De plus, dans un mode particulier de mise en oeuvre du procédé de l'invention, il est possible de synthétiser directement "in situ" le peracide en utilisant un mélange d'eau oxygénée et d'acide correspondant. Dans ce cas, l'acide peut être utilisé en quantités catalytiques, puisque, au cours de la réaction du peracide sur le pyronène, l'acide de départ est régénéré, et peut être recyclé en peracide en réagissant avec l'eau oxygénée.

En ce qui concerne l'eau oxygénée, elle peut être utilisée seule, en milieu basique, ou en combinaison avec :

– un métal ou,

– un nitrile (réaction de Radziszewski : Wiberg, J. Amer.Chem. Soc., 75, 3961 (1953)).

Parmi les métaux qui conviennent dans le procédé lorsque l'on utilise l'eau oxygénée, on peut citer les métaux de transition tels que notamment le tungstène, le molybdène, le vanadium, le titane, le platine ou le manganèse, éventuellement associés à un autre métal, comme par exemple l'étain.

Préférentiellement, on utilise le tungstène, le molybdène ou le platine.

Dans le cas des hydroperoxydes, on utilise comme agent époxydant l'association ROOH + Métal dans laquelle R est un radical alkyl et le métal est choisi parmi les métaux de transition tel le vanadium, le titane, le molybdène, le platine ou le cobalt. Préférentiellement, l'hydroperoxyde a pour formule :

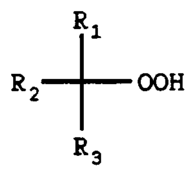

dans laquelle R1 à R3, identiques ou différents, représentent :

– des atomes d'hydrogène,
– des groupements alkyles linéaires ou ramifiés qui comportent de 1 à 30 atomes de carbone,
– des groupements cycloalkyles qui comportent de 3 à 12 atomes de carbone, ou
– des groupements alkyl- ou cycloalkylaromatiques qui comportent de 7 à 30 atomes de carbone.

Parmi les métaux utilisables lorsque l'agent epoxydant est un hydroperoxyde d'alkyle, on préfère le vanadium et le titane.

Dans le cas des acides hypohalogéneux, on utilise avantageusement les agents époxydants de formule X-OH dans laquelle X peut être Cl, Br ou I. Il est également possible de mettre en oeuvre le procédé de l'invention en utilisant tout précurseur de ces acides, et notamment les précurseurs générateurs d'ions halogènes (+). Il peut s'agir en particulier d'hypochlorite ou d'hypobromite de sodium ou de potassium à pH légèrement acide, ou d'ions Cl ou Br en présence d'eau et à pH légèrement acide, ou encore de dérivés Nh-alogénés d'amides tels que le N-bromosuccinimide ou le N-bromoacétamide également en présence d'eau.

Parmi les percarbonates et les perborates utilisables dans la présente invention, on peut citer notamment le percarbonate de sodium (Chem Letters 665 (1986)), le perborate de sodium (Tetrahedron Letters, 2967 (1988)), et les perborates d'alkyles (FR 1,447,267), dont l'effet sur l'époxydation des alcènes a été décrit.

La réaction d'époxydation est effectuée dans une atmosphère inerte, en présence d'un solvant choisi parmi :

– l'eau,
– les solvants étherés tels que l'éther éthylique ou propylique, le THF, ou encore le méthylterbutylether,
– les solvants halogénés tels que notamment les chlorobenzènes, le chloroforme, le chlorure de méthylène ou le dichloropropane,
– les hydrocarbures aliphatiques ou aromatiques et notamment les alcanes ayant plus de 5 atomes de carbone (hexane, heptane),
– les acides organiques, tels que l'acide acétique ou l'acide formique,
– les alcools, ou
– les esters.

Les différents réactifs peuvent être introduits simultanément mais on préfère ajouter l'agent époxydant sur le pyronène dissout dans le solvant.

Par ailleurs, il est possible d'ajouter au milieu un agent de transfert de phase pour faire de la catalyse par transfert de phase. Notamment, on peut ajouter:

– des sels d'ammonium quaternaire tels que l'hydroxyde, le bromure ou le chlorure de tétrabutylammonium, le chlorure de methyltrioctylammonium, ou le chlorure de diméthyl[dioctadécyl + dihexadécyl]ammonium;
– des hydrocarbures aromatiques ou chlorés;
– des sels de phosphonium, tels que notamment le chlorure d'hexadecyltributylphosphonium;
– certains complexes anioniques comme le tetrahexylammonium tetra(diperoxotungsto)phosphate.

Avantageusement, la réaction est effectuée en atmosphère inerte, à une température comprise entre -30°C et +100°C, et de préférence entre 0°C et 50°C. Il peut être particulièrement avantageux d'opérer à une température proche de la température ambiante.

Les conditions réactionnelles (température, nature du solvant et de l'agent époxydant, durée de la réaction) sont adaptées par l'homme de l'art à la vitesse optimale de réaction désirée et au produit recherché.

En particulier, l'utilisation d'acide hypohalogéneux permet généralement de former le monoépoxyde (1a), tandis que l'utilisation d'un peracide conduit majoritairement au diépoxyde (1b).

De même, le rapport molaire agent époxydant/pyronène est avantageusement compris entre 0,5 et 1,5 lorsque l'on veut préparer le composé (1a), et entre 1,5 et 2,5 lorsque l'on veut préparer le composé (1b).

Lorsque les deux produits sont obtenus simultanément, ils peuvent être séparés, par exemple par chromatographie en phase liquide.

Par ailleurs, lors de la préparation des composés de l'invention, il se forme également l'époxyde de formule (2a) décrit par ErhenFreund (document précité). Celui-ci peut être séparé des composés de l'invention, par exemple par chromatographie en phase liquide.

Le pyronène de départ utilisé dans la présente invention peut être obtenu de différentes façons. En particulier, il peut être préparé à partir du myrcène selon le protocole suivant (demande de brevet française FR 9002724):

- mise en contact du myrcène avec le dioxyde de soufre en présence d'un inhibiteur de polymérisation, à une température comprise entre 60 et 100°C, pour former la myrcène sulfone,
- traitement de la myrcène sulfone en présence d'un acide fort contenant moins de 5 % d'eau pour former le sulfolène cyclique, et
- chauffage du sulfolène cyclique, éventuellement en présence d'un catalyseur basique pour former le pyronène.

Au cours de la seconde étape, on peut utiliser comme acide fort les acides alkyl, aryl ou halogénosulfoniques, les résines Nafions, l'acide perchlorique, l'acide sulfurique ou différents catalyseurs hétérogènes acides.

Les composés de l'invention peuvent être utilisés comme stabilisants pour PVC, ou dans la synthèse de résines ou polymères.

La présente invention sera plus complètement décrite à l'aide des exemples suivants qui doivent être considérés comme illustratifs et non limitatifs.

## EXEMPLE 1

Dans un ballon de 25 ml, on place 0,27 g (2 mmole) de δ-pyronène et 0,105 g de décane (étalon interne) dans 11 ml d'éther anhydre à 0°C. On additionne 0, 78 g (3,2 mmole) d'acide métachloroperbenzoïque à 72 %. On laisse le mélange réactionnel remonter à température ambiante, et on laisse sous agitation pendant 28 heures. Le diepoxyde formé est dosé par chromatographie phase vapeur avec étalonnage interne.

On obtient ainsi 0,11 g de diépoxyde, soit 33% de rendement. Le diepoxyde contient 2 isomères, dont les données physicochimiques sont les suivantes :

RMN1H($CDCL_3$)δ : 0.89 m (6H) ; 1.35 à 1.95 m (6H) ; 2.51 (2H) ; 2.64 (2H).

En RMN du $^{13}C$, on détecte la présence de deux isomères

### Premier isomère

RMN$^{13}C$ ($CDCl_3$)δ : 20.0 (CH2) ; 22.9 ($CH_3$) ; 24.2 ($CH_3$) ; 32.1 ($CH_2$) ; 34.9 (Q) ; 38.1 ($CH_2$) ; 48.6 ($CH_2$) ; 52.4 ($CH_2$) ; 56.4 (Q) ; 61.7 (Q). Masse m/Z (intensité relative en %) : 153 ($M^+$-$CH_3$, 9) ; 95 (100).

### Deuxième isomère

RMN$^{13}C$ ($CDCl_3$)δ : 20.2 ($CH_2$) ; 22.5 ($CH_3$) ; 24.5 ($CH_3$) ; 33.4 ($CH_2$) ; 35.1 (Q) ; 39.1 ($CH_2$) ; 46.7 ($CH_2$) ; 50.7 ($CH_2$) ; 57.9 (Q) ; 63.0 (Q). Masse m/Z (intensité relative en %) : 153 ($M^+$-$CH_3$, 5) ; 95 (100).

## EXEMPLE 2

Dans un ballon de 20 ml, on place 2,27 g (0,013 mole) de N-bromosuccinimide dans un mélange acétone/eau : 9,4 ml/1,9 ml, sous $N_2$ à 0°C. On ajoute 1,5 g (0,011 mole) de δ-pyronène et laisse dissoudre. Lorsque tout le N-bromosuccinimide est dissout, on extrait à l'éther, lave avec une solution saturée de bicarbonate et sèche sur $MgS0_4$. Après évaporation des solvants, le brut réactionnel est dissout dans 10 ml de méthanol absolu, et 3,8 g de carbonate de potassium (2,5 équivalents) sont introduits à 0°C sous $N_2$.

Après 45 minutes sous agitation, le milieu est extrait à l'éther, on lave avec une solution HCl 1N puis avec une solution saturée de chlorure d'ammonium jusqu'à neutralité. Après évaporation des solvants, le brut réactionnel est purifié sur colonne d'alumine désactivée par élution avec une solution éther de pétrole/éther : 9/1.

on obtient ainsi 503 mg de monoépoxyde de formule:

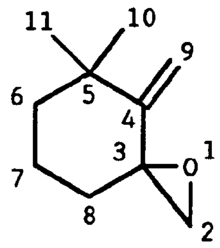

ayant les caractéristiques physicochimiques suivantes :
RMN[1]H ($CDCl_3$)δ : 1.06 S (3H) ; 1.13 S (3H) ; 1.44 à 1.80 m (6H) ; 3.28 à 3.64 (2H) ; 4.74 à 4.96 (2H).
RMN[13]C ($CDCl_3$)δ : 154.5 (C-4) ; 59.0 (C-3) ; 35.0 (C-8) ; 20.8 (C-7) ;
40.3 (C-6) ; 38.0 (C-5) ; 103.9 (C-9) ; 27.1 (C-10) ; 28.5 (C-11) ;
57.0 (C-2).
Masse m/Z (intensité relative en %) : 152 (M+,1.5) ; 84 (100).

## Revendications

**1 -** Composé de formule générale (1)

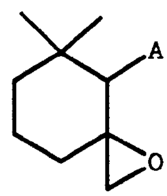

(1)

dans laquelle A représente un groupement méthylène (1a) ou oxirane (1b).

**2 -** Procédé de préparation d'un composé selon la revendication 1 caractérisé en ce que l'on effectue l'époxydation du pyronène de formule ( 3 ) :

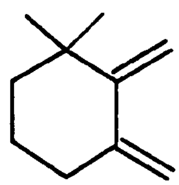

(3)

au moyen d'un agent époxydant.

**3 -** Procédé selon la revendication 2 caractérisé en ce que l'agent époxydant est choisi parmi les peracides ou leurs dérivés, les acides hypohalogéneux ou leurs précurseurs, l'eau oxygénée, les hydropéroxydes d'alkyles, les perborates et les percarbonates.

**4 -** Procédé selon la revendication 3 caractérisé en ce que le peracide est un acide ou un dérivé d'acide carboxylique aliphatique ou aromatique éventuellement substitué.

**5 -** Procédé selon la revendication 4 caractérisé en ce que le peracide est choisi parmi les acides peracétique, performique, perpropionique, pertrifluoroacétique, paranitroperbenzoïque, et métachloroperbenzoïque.

**6 -** Procédé selon l'une des revendications 3 à 5 caractérisé en ce que le peracide est formé "in situ".

**7 -** Procédé selon la revendication 3 caractérisé en ce que l'eau oxygénée est utilisée seule, en milieu basique, ou combinée à un nitrile ou à un métal, et de préférence à un métal de transition, éventuellement associé à un autre métal.

**8 -** Procédé selon la revendication 3 caractérisé en ce que l'hydroperoxyde consiste en la combinaison ROOH/métal dans laquelle R est un radical alkyl et le métal est un métal de transition.

**9 -** Procédé selon la revendication 8 caractérisé en ce que l'hydroperoxyde a pour formule :

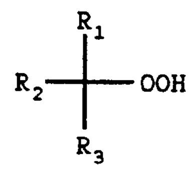

dans laquelle R1 à R3, identiques ou différents, représentent :

– des atomes d'hydrogène,
– des groupements alkyles linéaires ou ramifiés qui comportent de 1 à 30 atomes de carbone,
– des groupements cycloalkyles qui comportent de 3 à 12 atomes de carbone, ou
– des groupements alkyl- ou cycloalkylaromatiques qui comportent de 7 à 30 atomes de carbone.

**10 -** Procédé selon la revendication 7 caractérisé en ce que le métal de transition est choisi parmi le tungstène, le molybdène, le vanadium, le titane, le platine et le manganèse.

**11-** Procédé selon la revendication 8 caractérisé en ce que le métal de transition est choisi parmi le molybdène, le vanadium, le titane, le platine et le cobalt.

**12 -** Procédé selon la revendication 10 caractérisé en ce que le métal est choisi parmi le tungstène, le molybdène et le platine.

**13 -** Procédé selon la revendication 11 caractérisé en ce que le métal est choisi parmi le vanadium et le titane.

**14 -** Procédé selon l'une quelconque des revendications 2 à 13 charactérisé en ce que la réaction est effectuée en atmosphère inerte, à une température comprise entre -30°C et +100°C, et de préférence entre 0°C et 50°C.

**15 -** Procédé selon l'une quelconque des revendications 2 à 14 caractérisé en ce que, pour former préférentiellement le composé (Ia), le rapport molaire agent époxydant/pyronène est compris entre 0,5 et 1,5.

**16 -** Procédé selon l'une des revendications 2 à 14 caractérisé en ce que, pour former préférentiellement le composé (1b), le rapport molaire agent époxydant/pyronène est compris entre 1,5 et 2,5.

Office européen des brevets

# RAPPORT DE RECHERCHE EUROPEENNE

Numero de la demande

EP 91 40 2848

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | GB-A-1 098 884 (THE DUNLOP COMPANY LIMITED)<br>* le document en entier *<br>--- | 1-6 | C07D303/04<br>C07D493/10<br>// (C07D493/10, 303:00, 303:00) |
| D,A | HELVETICA CHIMICA ACTA<br>vol. 57, no. 4, 5 Juin 1974, BASEL CH<br>pages 1098 - 1116;<br>J. EHRENFREUD ET AL.: 'Oxydation von Allylalkoholen mit Blei(IV)-acetat'<br>* le document en entier, et en particulier page 1099, composé 2 *<br>----- | 1 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| LA HAYE | 17 JANVIER 1992 | ALLARD M.S. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)